# EUROPEAN PATENT SPECIFICATION

(11) **EP 2 331 167 B1**
(45) Date of publication and mention of the grant of the patent: **17.08.2016**
(21) Application number: 09817876.7
(22) Date of filing: 25.09.2009
(51) Int. Cl.: A61M 5/14, A61M 5/158, A61M 25/06, A61M 5/32

(54) **ANTI-NEEDLESTICK SYSTEM**
NADELSTICHSCHUTZSYSTEM
SYSTÈME ANTI-BÂTONS À ÉPINGLES

(30) Priority: 01.10.2008 US 286581
(43) Date of publication of application: 15.06.2011
(73) Proprietor: Kawasumi Laboratories, Inc., Oita 876-0121 (JP)
(72) Inventor: HASHIMOTO, Kazuhiko, Tampa, Florida 33623-4355 (US); PAVLO, John, A., Tampa, Florida 33623-4355 (US)
(74) Representative: Hering, Hartmut
(86) International application number: PCT/JP2009/067204
(87) International publication number: WO 2010/038844

(56) References cited:
- WO-A1-02/30491
- WO-A1-2005/105195
- WO-A1-2012/032378
- JP-A- 7 184 997
- US-A- 3 610 240
- US-A- 5 120 320
- US-A- 5 147 319
- US-A- 5 266 072
- US-A1- 2003 181 874
- US-B1- 6 595 965

## Description

### Technical Field

The present invention relates to an anti-needlestick system and more particularly pertains to shielding a needle immediately as it is withdrawn from a patient, the shielding being in a safe, convenient and economical manner.

### Background Art

The use of needle systems of known designs and configurations is known in the prior art. More specifically, needle systems of known designs and configurations previously devised and utilized for the purpose of shielding needles through known methods and apparatuses are known to consist basically of familiar, expected, and obvious structural configurations, notwithstanding the myriad of designs encompassed by the crowded prior art which has been developed for the fulfillment of countless objectives and requirements.

By way of example, U. S. Patent Number 5,266,072 issued November 30, 1993 to Utterberg relates to a Guarded Winged Needle Assembly.

While this device fulfills particular objectives and requirements, the aforementioned patent does not describe an anti-needlestick system that allows for shielding a needle immediately as it is withdrawn from a patient, the shielding being in a safe, convenient and economical manner.

In this respect, the anti-needlestick system according to the present invention substantially departs from the conventional concepts and designs of the prior art, and in doing so provides an apparatus primarily developed for the purpose of shielding a needle immediately as it is withdrawn from a patient, the shielding being in a safe, convenient and economical manner.

Therefore, it can be appreciated that there exists a continuing need for a new and improved anti-needlestick system which can be used for shielding a needle immediately as it is withdrawn from a patient, the shielding being in a safe, convenient and economical manner. In this regard, the present invention substantially fulfills this need.

Conventionally, there are known several types of anti-needlestick system, among which, most typical one deals with a winged needle assembly in which a winged needle is extended from its needle cover when in use and retreated into the cover or sheath after use and held there safe for disposal. US 5 120 320 A and US 6 595 965 B1 belong to this category. The other type is "post cover" type winged needle assembly, in which a needle cover or sheath is attached to the winged needle and cover it after the needle is withdrawn from a patient's arm. US 2003/181874 A1, JP 7 184997 A, and WO 02/30491 A1 belong to this category. Still other type is a "winged sheath" type assembly wherein bare needle without wings is used and then held in the sheath with a specific fixing mechanism as disclosed by WO 2005/1051954 A1. The winged sheath type is also practiced as a "post-cover" type winged sheath assembly as disclosed in US 5 147 319 A.

### Disclosure of Invention

### Technical Problem

In view of the foregoing disadvantages inherent in the known types of needle systems of known designs and configurations now present in the prior art, the present invention provides an improved anti-needlestick system. As such, the general purpose of the present invention, which will be described subsequently in greater detail, is to provide a new and improved anti-needlestick system and method which has all the advantages of the prior art and none of the disadvantages.

It is therefore an object of the present invention to provide a new and improved anti-needlestick system which has all of the advantages of the prior art needle systems of known designs and configurations and none of the disadvantages.

It is another object of the present invention to provide a new and improved anti-needlestick system which may be easily and efficiently manufactured and marketed.

It is further object of the present invention to provide a new and improved anti-needlestick system which is of durable and reliable constructions.

An even further object of the present invention is to provide a new and improved anti-needlestick system which is susceptible of a low cost of manufacture with regard to both materials and labor, and which accordingly is then susceptible of low prices of sale to the consuming public, thereby making such anti-needlestick system economically available to the buying public.

Even still another object of the present invention is to provide an anti-needlestick system for shielding a needle immediately as it is withdrawn from a patient, the shielding being in a safe, convenient and economical manner.

Lastly, it is an object of the present invention to provide a new and improved anti-needlestick system. A housing assembly has a body portion. The body portion has distal and proximal ends, a cylindrical extent, and upper and lower sections. The distal end has a first aperture. The proximal end has a second aperture. The upper section has a lower edge and the lower section has an upper edge. A pair of flexible hinge portions is provided. The upper ends are coupled to the upper section. The lower ends are coupled to the lower section. The hinge portions are adapted to allow movement between closed and open orientations. Handling elements are formed with the main body portion. The handling elements include an upwardly extending projection located on the upper section essentially coplanar with the distal end to facilitate one handed utilization of the system.

### Technical Solution

To attain this, the present invention essentially comprises an anti-needlestick system. First provided is a housing assembly. The housing assembly has a main body portion. The main body portion has a distal end. The distal end has a first aperture. The main body portion has a proximal end. The proximal end has a second aperture. The main body portion has a cylindrical extent. The cylindrical extent is in a generally rectangular exterior cross sectional configuration over the majority of its length between the distal and proximal ends. The main body portion has a semi-cylindrical upper section. The upper section has a lower edge. The upper section has a semi-cylindrical lower section. The lower section has an upper edge. The main body section has a pair of flexible hinge portions. The upper ends of the hinge portions are coupled to the upper section at the distal end. The lower ends of the hinge portions are coupled to the lower section at the distal end. The hinge portions are adapted to allow movement of the halves between a closed operative orientation and an open inoperative orientation. In this closed operative orientation the upper and lower edges are provided in contact in a horizontal plane. In this manner the main body portion is formed into a cylinder with a circular cross section. In the open inoperative orientation the upper and lower edges are provided out of contact and spaced from each other. The hinge portions are located on opposite sides of the first aperture.

Handling elements are provided. The handling elements are formed with the main body portion. The handling elements include an upwardly extending flange projection. The upwardly extending flange projection is provided on the upper section coplanar with the distal end. The handling elements include a plurality of laterally extending non-slip recesses. The laterally extending non-slip recesses are provided on the upper section proximally of the upwardly extending flange projection. The handling elements also include outwardly extending female projections. The outwardly extending female projects are provided on the upper section adjacent to the proximal end. The handling elements include male projections. The male projections extend upwardly from the lower section. The male projections are provided in a co-operable relationship with the female projection. In this manner the sections are releasably secured together in the closed orientation.

Provided next is a needle assembly. The needle assembly has a cylindrical hollow hub. The hub has a distal end. The hub has a proximal end. The hub is slidably positioned within the housing assembly. The hub has flat laterally extending wings. The needle assembly includes a needle. The needle is coupled to the distal end of the hub. The needle is slidably received in the first aperture. The needle assembly includes a flexible tube. The flexible tube is coupled to the proximal end of the hub. The flexible tube is slidably received in the second aperture. The needle assembly includes a cover. The cover is removably positioned over the needle when in the extended orientation.

Provided last are guides. The guides are formed in the edges of the sections. The guides include a primary recess. The primary recess is formed in the upper edge of the lower section between a forward end proximally of the upwardly extending flange projection and a rearward end distally of the male and female projections. The guides include a forward recess. The forward recess is formed in the lower edge of the upper section proximally of the forward end of the primary recess. The guides also include an abutment surface. The abutment surface is formed in the lower edge of the upper section distally of the rearward end of the primary recess. The wings are adapted to extend laterally from the hub through the primary recess at an extended orientation. The majority of the needle extends distally of the main body portion. The forward recess assists in retaining the needle in the extended orientation. The wings are adapted to extend laterally from the hub through the primary recess at a retracted orientation. In the retracted orientation the entirety of the needle is provided totally within the main body portion. The abutment surface assists in retaining the needle in the retracted orientation. The needle assembly includes the needle. The needle is adapted to be moved from the extended orientation to the retracted orientation by a user sliding the wings within the primary recess.

There has thus been outlined, rather broadly, the more important features of the invention in order that the detailed description thereof that follows may be better understood and in order that the present contribution to the art may be better appreciated. There are, of course, additional features of the invention that will be described hereinafter and which will form the subject matter of the claims attached.

In this respect, before explaining at least one embodiment of the invention in detail, it is to be understood that the invention is not limited in its application to the details of construction and to the arrangements of the components set forth in the following description or illustrated in the drawings. The invention is capable of other embodiments and of being practiced and carried out in various ways. Also, it is to be understood that the phraseology and terminology employed herein are for the purpose of descriptions and should not be regarded as limiting.

The objects of the invention, along with the various features of novelty which characterize the invention, are defined by the claims annexed to and forming a part of this disclosure. For a better understanding of the invention, its operating advantages and the specific objects attained by its uses, reference should be made to the accompanying drawings and descriptive matter in which there are illustrated preferred embodiments of the invention.

### Advantageous Effects

According to the present invention it is provided a new and improved anti-needlestick system which is easily and efficiently manufactured and marketed.

According to the present invention it is also provided a new and improved anti-needlestick system which is of durable and reliable constructions.

According to the present invention it is also provided a new and improved anti-needlestick system which is susceptible of a low cost of manufacture with regard to both materials and labor, thereby making such anti-needlestick system economically available to the buying public.

According to the present invention it is also provided an anti-needlestick system for shielding a needle immediately as it is withdrawn from a patient, the shielding being in a safe, convenient and economical manner.

### Brief Description of the Drawings

Figure 1 is a plan view of an anti-needlestick system constructed in accordance with the principles of the present invention.
Figure 2 is a side elevational view of the system taken along line 2-2 of Figure 1.

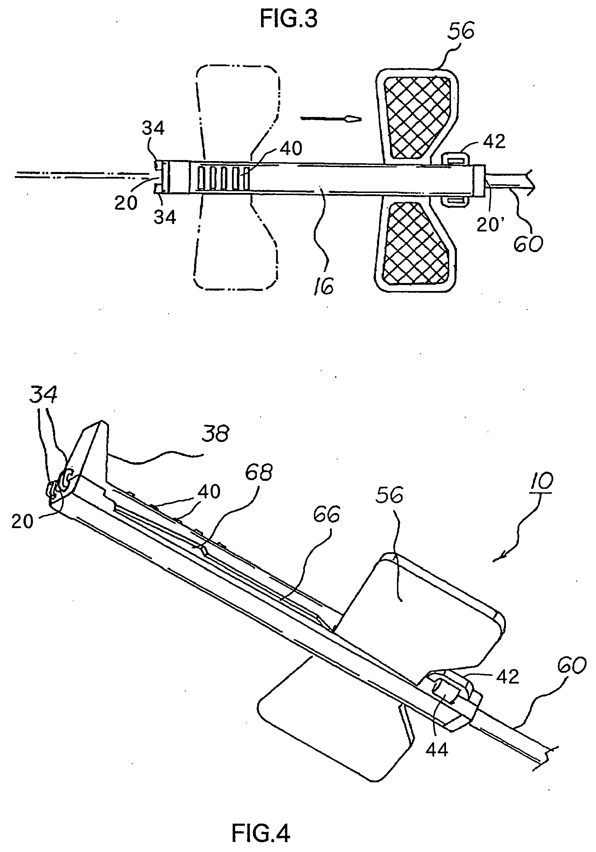

Figure 3 is a plan view similar to Figure 1 but with the needle assembly in the retracted orientation.
Figure 4 is a perspective illustration of the system shown in the prior Figures.

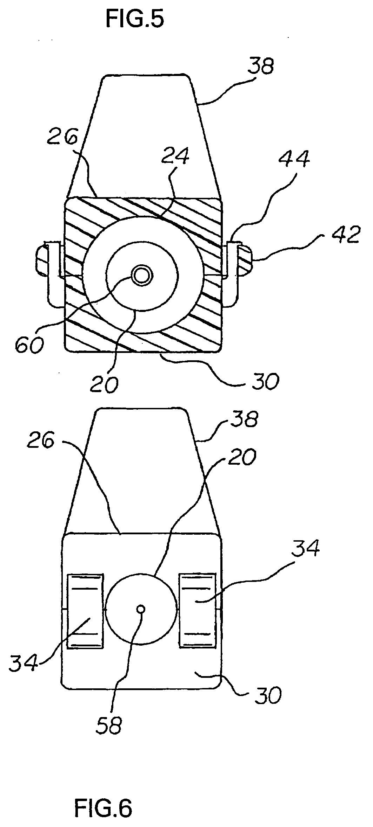

Figure 5 is a cross sectional view taken along line 5-5 of Figure 2.
Figure 6 is a front elevation view taken along line 6-6 of Figure 2.

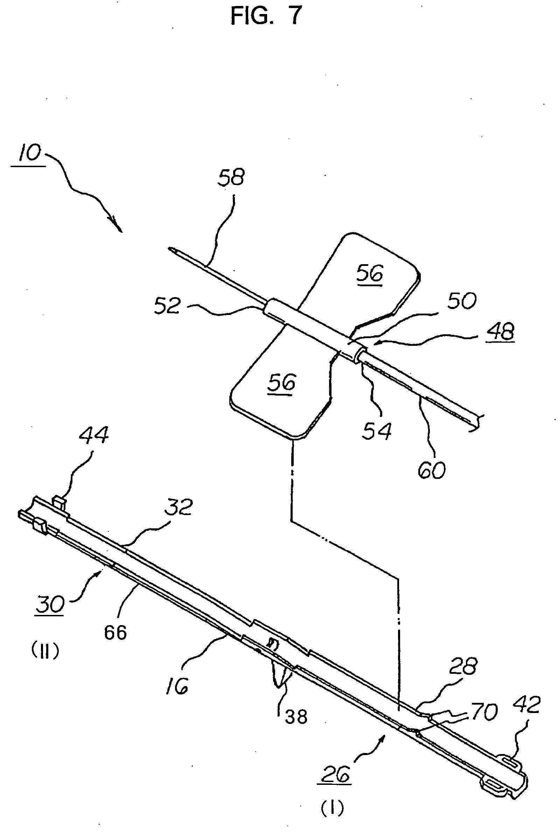

Figure 7 is an exploded perspective illustration of the system shown in the prior Figures.

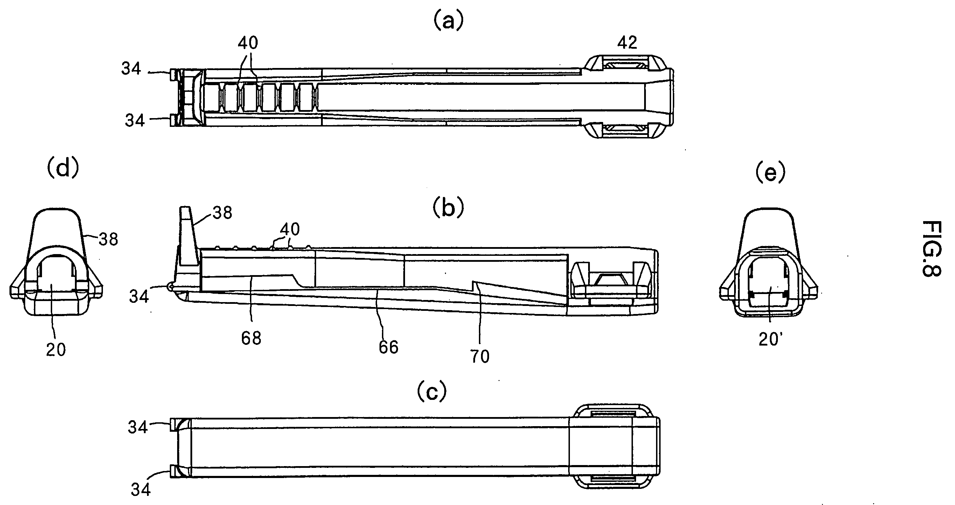

Figure 8 is a six-sided illustration of the housing assembly comprising the anti- needlestick system in a closed orientation. Wherein (a) is a plan view, (b) is a side elevation view. (c) is a bottom end view. (d) is a left side view. (e) is a right side view.

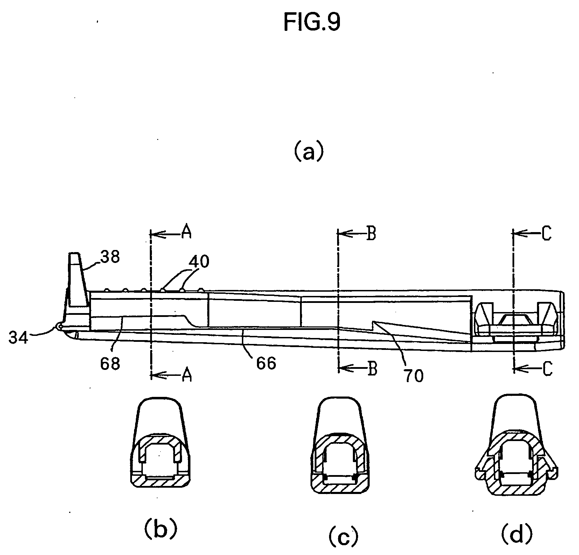

Figure 9 (a) is a side elevation view of the housing assembly comprising the anti- needlestick system in a closed orientation. Figure (b)-(d) are cross sectional views taken along line A-A, B-B, and C-C of the Figure 9 (a) respectively.

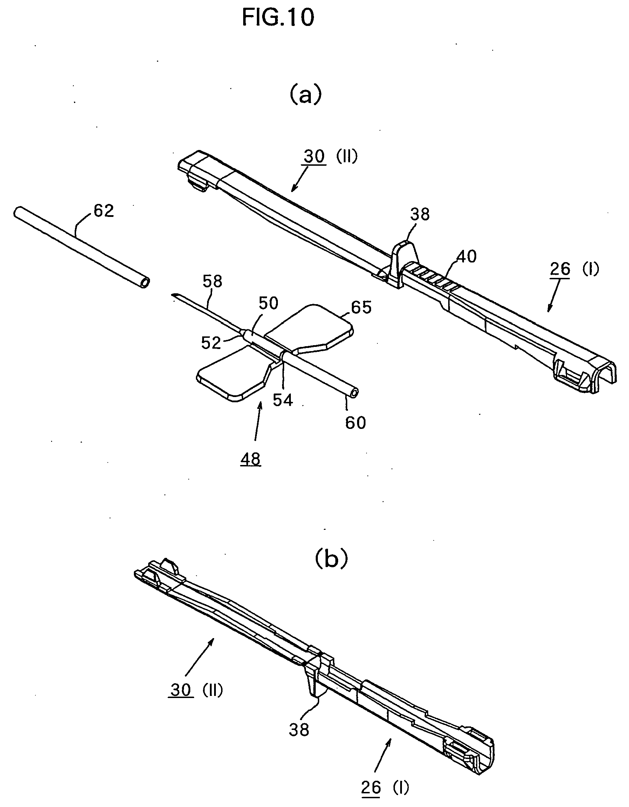

Figure 10 is a perspective illustration of the way the anti-needlestick system is constructed, wherein (a) is a up-sided view and (b) is a bottom end view of the housing assembly in a coplanar or full open orientation.

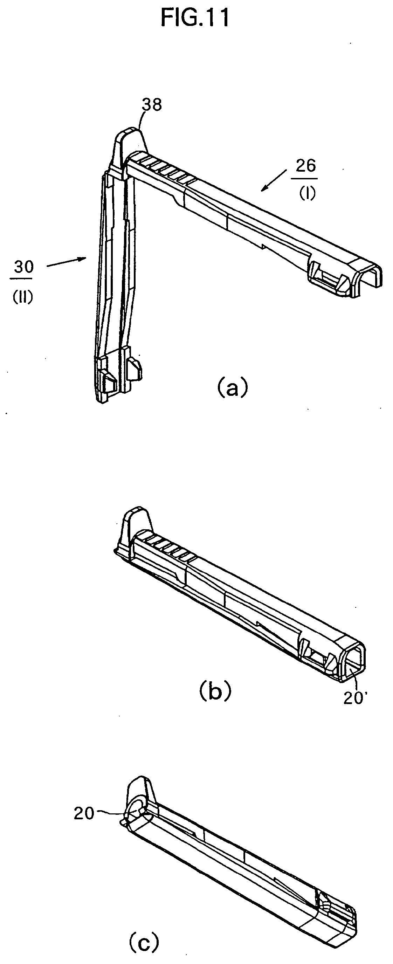

Figure 11 is a perspective illustration of the way the anti-needlestick system is constructed, wherein (a) is perspective view of the housing assembling in an half open orientation, (b) is the perspective up-side view of the housing assembly in closed orientation and (c) is a perspective bottom end view of the housing assembly in closed orientation.

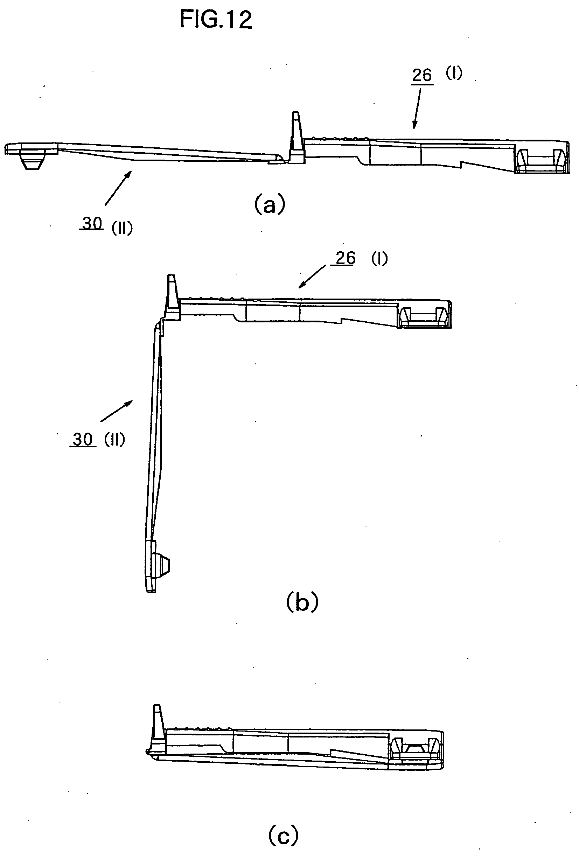

Figure 12 is a perspective illustration of the way the anti-needlestick system is constructed, wherein (a) is a side elevation view of the housing assembly wherein upper section I and lower section II are in an open and in a coplanar position corresponding to that of Figure 7, (b) is the side elevation view of the housing assembly wherein upper section I and lower section II are in a half open orientation approaching with each other and (c) in the side elevation view wherein upper section I and lower section II are engaged with each other and form a entirely closed orientation.

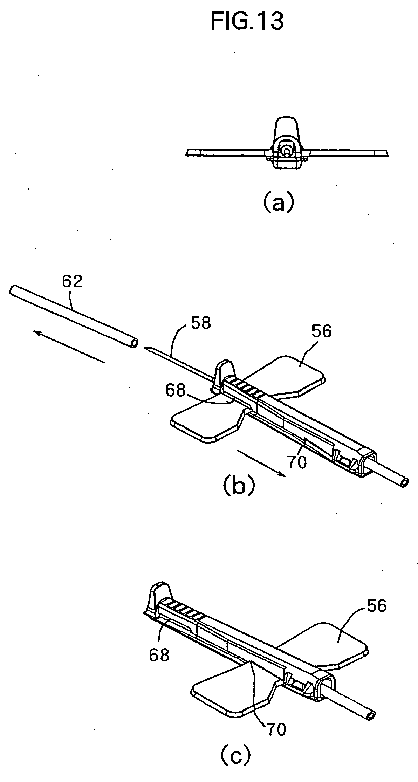

Figure 13 is a perspective illustration of the way the anti-needlestick system is constructed, wherein (a) is a side view of the anti-needlestick system wherein needle is in an extended orientation, (b) is the perspective view of the system wherein cover 62 is being removed from the needle 58 and (c) is the perspective view wherein needle is in a retreated orientation and the system is in a closed orientation.

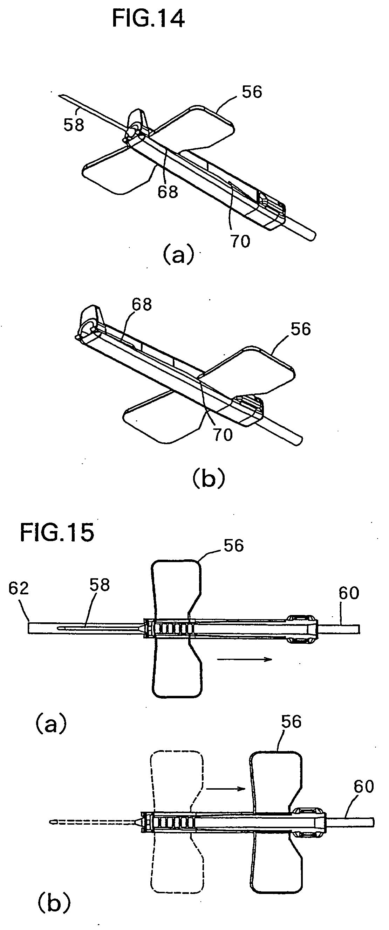

Figure 14 is a perspective illustration of the extended and retreated orientation, wherein (a) is a perspective bottom side view of the system in an extended orientation and (b) is a perspective bottom side view of the system in an retreated orientation.
Figure 15 is a planar illustration of the extended and retreated orientation, wherein (a) is a plan view of the system in an extended orientation and (b) is a plan view in an retreated orientation.
Figure 16 is a perspective illustration of the upwardly extending flange projection wherein (a) is a perspective view of the system, (b) is a side elevation view of the system and (c) is an partly enlarged side-elevation view corresponding to the part E shown in Figure 16(b), showing angles *θ* 1 and *θ* 2.
Figure 17 is a perspective illustration of the laterally existing non-slip recess 40, wherein (a) is a perspective view of the system and (b) is a partly enlarged perspective view corresponding to the part E shown in Figure 17(a).
Figure 18 is a planar illustration of the bottom floor 75 configuration of the system having an angle *θ* with respect to line L parallel to needle 58, wherein (a) is a side elevation view of the system having an angle *θ*, (b) is a side elevation view of the system when placed on a planar surface L' such as a patient skin and (c) is a partly enlarged side elevation view of the figure 18(b).

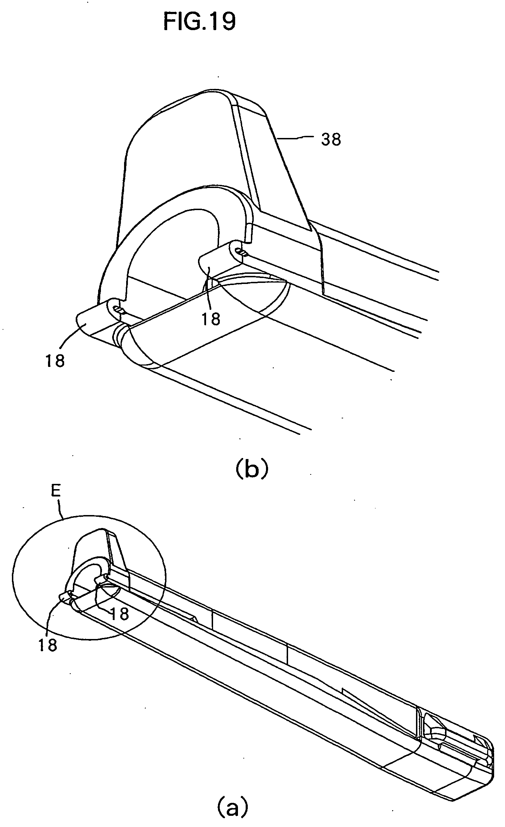

Figure 19 is a perspective illustration of the hinge portion 18 wherein (a) is a perspective bottom end view of the system and (b) is a partly enlarged perspective view of the portion E in figure 19(b).
Figure 20 is a perspective illustration of the system showing that the system has a "chamfered constitution" wherein most edges and corners are chamfered constitution and have round chamfered finishing r1 r2, r3, r4, r5, r6, r7,....

### Explanation of Reference Numerals

- 10: anti-needlestick system
- 14: housing assembly
- 48: needle assembly
- 16: main body portion
- 18: distal end
- 20: first aperture
- 22': second aperture
- 22: proximal end
- 24: generally cylindrical extent
- 26: semi-cylindrical upper section
- 28: lower edge
- 30: semi-cylindrical lower section
- 32: upper edge
- 34: hinge portions
- 38: upwardly extending flange projection
- 40: laterally extending non-slip recesses
- 42: outwardly extending female projections
- 44: male projections
- 48: needle assembly
- 50: cylindrical hollow hub
- 52: distal end
- 54: proximal end
- 56: wings
- 58: needle
- 60: flexible tube
- 62: cover
- 66: primary recess
- 68: forward recess
- 70: abutment surface

### Best Mode for Carrying Out the Invention

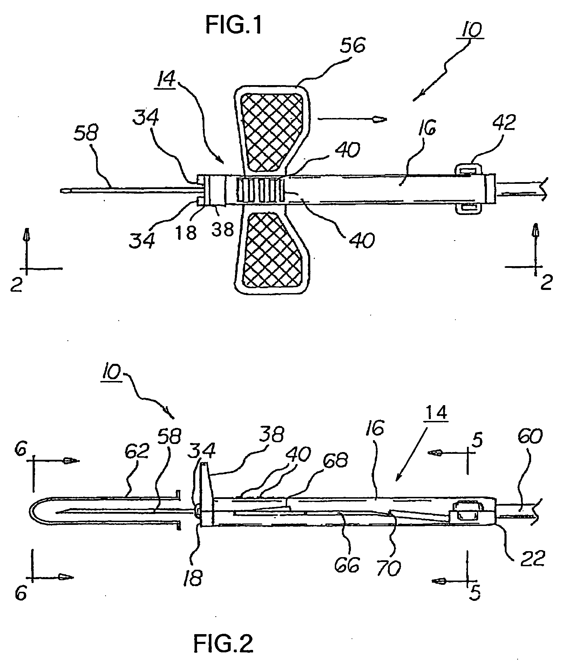

The invention will be better understood and objects other than those set forth above will become apparent when consideration is given to the following detailed description thereof. Such description makes reference to the annexed drawings. With reference now to the drawings, and in particular to Figure 1 thereof, the preferred embodiment of the new and improved anti-needlestick system embodying the principles and concepts of the present invention and generally designated by the reference numeral 10 will be described.

The present invention, the anti-needlestick system 10 is comprised of a plurality of components. Such components in their broadest context include a housing assembly 14 and a needle assembly 48. Such components are individually configured and correlated with respect to each other so as to attain the desired objective.

### (housing assembly)

First provided is a housing assembly 14 (or also called a needle cover). The housing assembly 14 has a main body portion 16. The main body portion 16 has a distal end 18. The distal end 18 has a first aperture 20. The main body portion 16 has a proximal end 22. The proximal end 22 has a second aperture 20'. The main body portion 16 has a generally cylindrical extent 24. The generally cylindrical extent 24 is in a generally rectangular exterior cross sectional configuration over the majority of its length between the distal and proximal ends. The main body portion 16 has a semi-cylindrical upper section 26 or I. The upper section 26 has a lower edge 28. The upper section 26 is coupled to a semi-cylindrical lower section 30 or II. The lower section 30 has an upper edge 32.

### (hinge portions, closed and open orientations)

The main body section 16 has a pair of flexible hinge portions 34. The upper ends of the hinge portions 34 are coupled to the upper section at the distal end 18. The lower ends of the hinge portions 34 are coupled to the lower section at the distal end 18. The hinge portions are adapted to allow movement of the halves between a closed operative orientation (COO) and an open inoperative orientation (OIO). In this closed operative orientation (COO) the upper and lower edges 32, 28 are provided in contact in a horizontal plane. In this manner the main body portion 16 is formed into a cylinder with a generally circular cross section. In the open inoperative orientation (OIO) the upper and lower edges 32, 28 are provided out of contact and spaced from each other. The hinge portions 34 are located on opposite sides of the first aperture 20 and form a living hinge.

### (handling elements)

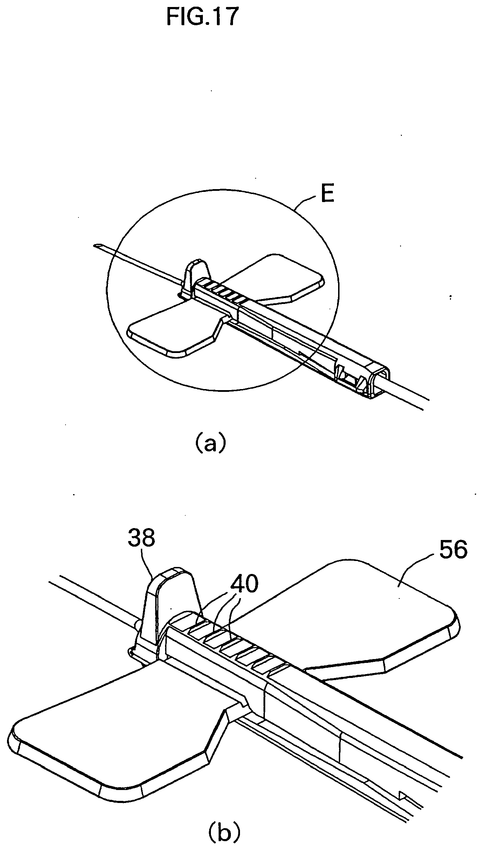

Handling elements H are provided. The handling elements are formed with the main body portion 16. The handling elements H include an upwardly extending flange projection 38. The upwardly extending flange projection 38 is provided on the upper section coplanar with the distal end 18. The handling elements H include a plurality of laterally extending non-slip recesses 40. The laterally extending non-slip recesses 40 are provided on the upper section proximally of the upwardly extending flange projection 38. The detailed configuration of the laterally extending non-slip recesses 40 is illustrated in figure 17(b). The non-slip recesses 40 prevent accidental slipping of fingers when handling the assembly and impart increased handleability or operability to the medical practitioners in a similar fashion as with the flange projection 38, which is detailed as follows.

The handling elements H also include outwardly extending female projections 42. The outwardly extending female projects 42 are provided on the upper section adjacent to the proximal end. The handling elements H include male projections 44. The male projections 44 extend upwardly from the lower section. The male projections 44 are provided in a co-operable relationship with the female projection 42. In this manner the sections are releasably secured together in the closed orientation.

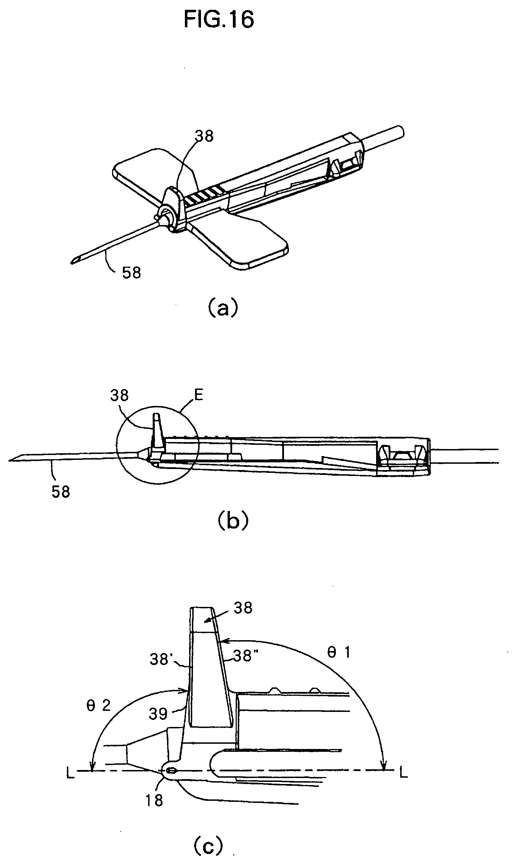

Referring to Figure 16, more detailed explanation about the function of upwardly extending flange projection 38 is given. The primary object of providing the flange projection 38 is to impart increased handleability or operability to a medical practitioner, who presses or hooks his or her thumb or index finger against the flange projection 38 and with this hold some operation such as withdrawal of needle or tube into the housing assembly is carried out without involving accidental needle sticking. In the invention, flange projection 38 is preferably constituted to rise at an angel greater than right angle (90° )thereby giving far improved feeling of fitness of the fingers and securing increased manipulability. The angle *θ*1 made between the face of flange projection 38 and floor line L is preferably given by 90° < *θ*1 < 120° as shown in Figure 16(c) (line L is a line parallel to the needle).

Additionally thus making up-rising flange projection angel *θ*1 greater than right angel contributes to greater mold releasability of this housing assembly when it is manufactured by injection molding procedure, wherein the angled-flange (or tapered-flange) projection functions as a "draft". The housing assembly is ordinarily made of hard or semi hard plastic materials such as hard polyvinyl chloride or polypropylene resin or the like using injection molding technology.

It is also preferable that the end portion (or end wall) 39 of the housing assembly is so constituted as to have an angle *θ*2 with respect to the floor line L greater than right angle, more preferably the angle is given by 90° < *θ*2 < 120°, as shown in Figure 16(c) to secure effective needle length, which is the maximum length extendable from the housing assembly to be exposed. When molding an article such as housing assembly which has an end portion, with hinge portion 18 at the center of it, having an end-portion angel *θ* 2 greater than right angel leads to greater mold releasability of the assembly when it is manufactured by injection molding procedure for the similar reason described above. In the polymer injection processing the angled-end (or tapered end) portion also facilitates good flow of resin in the mold and prevents the short shot of resin.

### (needle assembly)

Provided next is a needle assembly 48. The needle assembly 48 has a cylindrical hollow hub 50. The hub 50 has a distal end 52. The hub 50 has a proximal end 54. The hub 50 is slidably positioned within the housing assembly 14. The hub 50 has a flat laterally extending a pair of wings 56. The needle assembly 14 includes a needle 58. The needle 58 is coupled to the distal end 52 of the hub 50. The needle 58 is slidably received in the first aperture 20. The needle assembly 48 includes a flexible tube 60. The flexible tube 60 is coupled to the proximal end 54 of the hub 50. The flexible tube 60 is slidably received in the second aperture 20'. The needle assembly 48 includes a cover 62. The cover 62 is removably positioned over the needle 58 when in the extended orientation (EO).

### (guide)

Provided last are guides G. The guides G are formed in the edges of the sections. The guides G include a primary recess 66. The primary recess 66 is formed in the upper edge 32 of the lower section 30 or (II) between a forward end proximal of the upwardly extending flange projection 38 and a rearward end distal of the male and female 44,42 projections. The guides G include a forward recess 68. The forward recess 68 is formed in the lower edge of the upper section 26 or (I) proximally of the forward end of the primary recess 66. The guides G also include an abutment surface 70. The abutment surface 70 is formed in the lower edge of the upper section distal of the rearward end of the primary recess 66. The wings 56 are adapted to extend laterally from the hub 50 through the primary recess 66 at an extended orientation (EO). The majority of the needle 58 extends distal of the main body portion 16. The forward recess 68 assists in retaining the needle in the extended orientation (EO). The wings 56 are adapted to extend laterally from the hub 50 through the primary recess 66 at a retracted orientation (RO). In the retracted orientation the entirety of the needle 58 is provided totally within the main body portion 16. The abutment surface 70 assists in retaining the needle in the retracted orientation (RO). The needle assembly 48 includes the needle 58. The needle 58 is adapted to be moved from the extended orientation (EO) to the retracted orientation (RO) by a user sliding the wings within the primary recess.

### (characteristics of bottom floor)

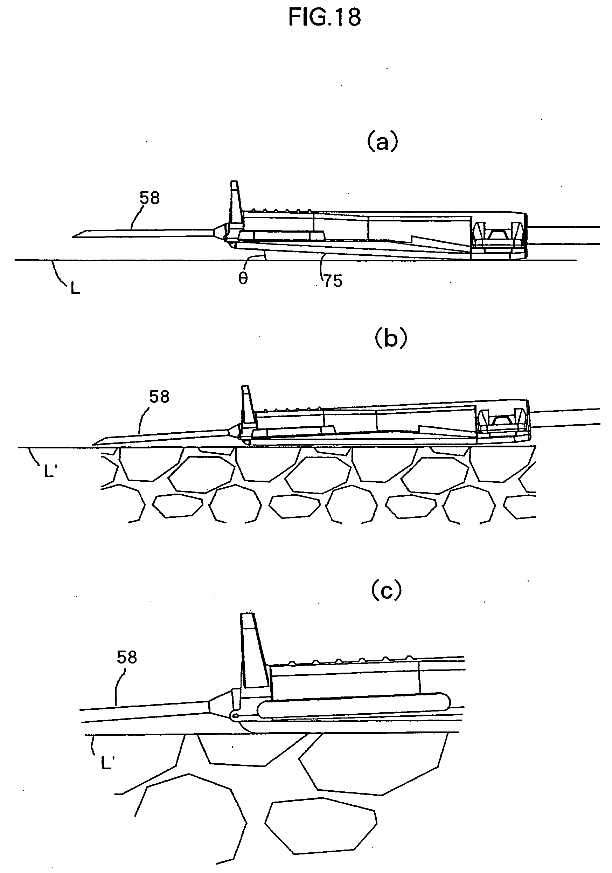

In the present invention as shown in figure 18, it is preferable that the bottom floor or bottom surface 75 of the housing assembly is so configured as to have an angle *θ* more than zero (0°) with respect to line L, which is parallel to the needle 58 (or the patient' s skin L'), the angle being preferably given by 0° < *θ*< 10° as shown in Figure 18(a) .

Thus having a configuration of the angled bottom floor line (ABFL) contributes to achieve mainly two objectives: The first is to keep or turn the needle-tip downwards, thereby securing a stable positioning of the anti-needlestick system while the needle is being punctured to the patient skin for medical treatment. The second is to effectively reduce the height or profile of the housing assembly thereby making this configuration of the assembly as compact as possible for helping the patient feel comfortable and easy while the assembly is fixed or attached onto his or her skin for treatment.

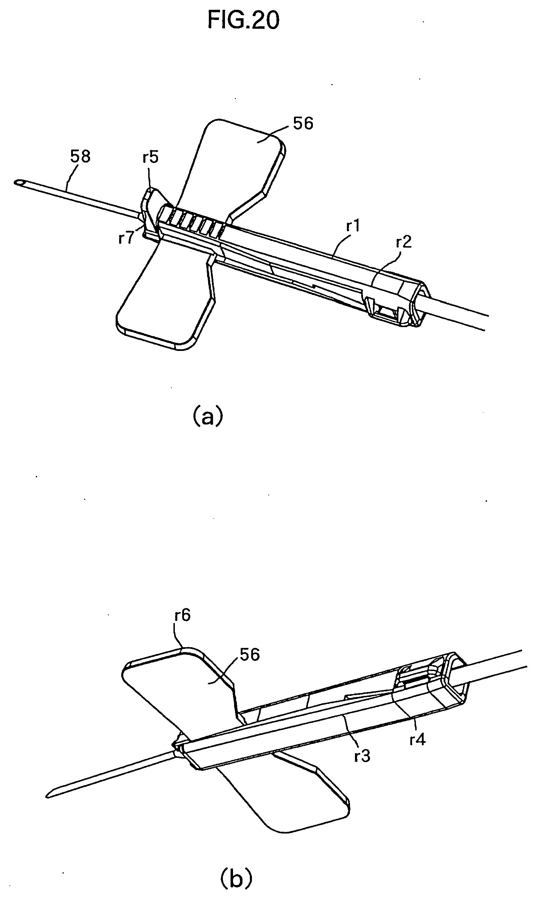

As shown in Figure 20, the anti-sticking system of the present invention has a chamfered constitution wherein most edges and corners are chamfered and have round chamfered finishing r1 r2, r3, r4, r5, r6, r7,... Usually a medical practitioner or a nurse handles the anti-sticking device with gloved hands for prevention of contagious infection. And the thus full chamfered constitution greatly contributes to reduce the chance of hang-up of gloved fingers with the devise. The full chamfering design also makes great contribution to improve the feelings of the patient and thereby eliminating his or her discomfort while the anti-sticking device is in use and its bottom side surface is put into direct contact with the patient's skin.

### (manner of usage of the system)

The components of the system of the present invention, particularly the upwardly extending flange projection 38, act to facilitate one-handed utilization of the system. Since the upwardly extending flange projection 38 is essentially coplanar with the distal end 18 of the housing 14, a user of the system may place an index finger against the projection while using a thumb and middle finger of the same hand to withdraw the needle from the patient. The other hand of the user is thus free to apply pressure to the patient at the injection site with gauze or cotton. Additionally, the rectangular cross section of the main body portion 16, with a planar section in contact with the patient's skin, acts to abate rocking of the system as during withdrawal of the needle.

As to the manner of usage and operation of the present invention, the same should be apparent from the above description. Accordingly, no further discussion relating to the manner of usage and operation will be provided.

With respect to the above description then, it is to be realized that the optimum dimensional relationships for the parts of the invention, to include variations in size, materials, shape, form, function and manner of operation, assembly and use, are deemed readily apparent and obvious to one skilled in the art, and all equivalent relationships to those illustrated in the drawings and described in the specification are intended to be encompassed by the present invention.

Therefore, the foregoing is considered as illustrative only of the principles of the invention. Further, since numerous modifications and changes will readily occur to those skilled in the art, it is not desired to limit the invention to the exact construction and operation shown and described, and accordingly, all suitable modifications and equivalents may be resorted to, falling within the scope of the invention.

### Industrial Applicability

The present invention directed to a new and improved anti-needlestick system which is easily and efficiently manufactured and marketed, can be preferably applied to the medical and healthcare field.

The present invention directed to a new and improved anti-needlestick system which is of durable and reliable constructions , can be preferably applied to the medical and healthcare field.

The present invention directed to a new and improved anti-needlestick system which is susceptible of a low cost of manufacture with regard to both materials and labor, thereby making such anti-needlestick system economically available to the buying public, can be preferably applied to the medical and healthcare field.

The present invention directed to an anti-needlestick system for shielding a needle immediately as it is withdrawn from a patient, the shielding being in a safe, convenient and economical manner, can be preferably applied to the medical and healthcare field.

## Claims

1. An anti-needlestick system (10) for shielding a needle of a winged-needle immediately as it is withdrawn from a patient using only one hand, the shielding being in a safe, convenient and economical manner, the system comprising,
(i) a housing assembly (14),
(ii) a handling element (H),
(iii) a needle assembly (48), and
(iv) guides (G),
working in combination , wherein
(i) the housing assembly (14) for accommodating the needle assembly (48) slidably placed in it;, having,
a main body portion (16) with a distal end (18) formed with a first aperture (20) and a proximal end (22) formed with a second aperture (20') and with a cylindrical extent in a generally rectangular exterior cross sectional configuration over the majority of its length between the distal and proximal ends (18,22),
the main body portion (16) having a semi-cylindrical upper section (I) with a lower edge (28) and a semi-cylindrical lower section (II) with an upper edge (32),
a pair of flexible hinge portions (34) with upper ends coupled to the upper section (I) at the distal end (18) and lower ends coupled to the lower section (II) at the distal end (18),
the hinge portions (34) being adapted to allow movement of the halves between a closed operative orientation and an open inoperative orientation,
the closed operative orientation being with the upper and lower edges in contact in a horizontal plane to form the main body portion (16) into a cylinder with a circular cross section,
the open inoperative orientation being with the upper and lower edges out of contact and spaced from each other, the hinge portions (34) being located on opposite sides of the first aperture;
(ii) the handling elements (H) for providing the system with good manipulation capability of allowing a one-hand needle withdrawal procedure, formed with the main body portion (16),
the handling elements (H) including an upwardly extending plate-like-shaped flange projection (38) located on the semi-cylindrical upper section (26) substantially coplanar with the distal end (18) of the main body portion (16),
the plate-like-shaped flange projection (38) having two sides comprising a forward-looking face (38') on the distal side and a backward-looking face (38") on the proximal side,
the forward-looking face of the flange-projection is substantially vertical and coplanar with the distal end (18) and
the backward-looking face is upwardly slanted at an angle greater than right angle (90°) and smaller than 120°or 90°< θ1 < 120° wherein θ1 is the angle made between the backward-looking face and floor line L,
the handling elements (H) including a plurality of laterally extending non-slip recesses (40) located on the upper section proximal of the upwardly extending flange projection (38),
the handling elements (H) also including outwardly extending female projections (42) located on the upper section adjacent to the proximal end with male projections (44) extending upwardly from the lower section in co-operable relationship with the female projection (42) for releasably securing the sections together in the closed orientation (CO);
(iii) the needle assembly (48) including,
cylindrical hollow hub (50) with a distal end (52) and a proximal end (54) slidably positioned within the housing assembly (14),
the hub (50) having flat laterally extending wings (56), a needle (58) coupled to the distal end (54) of the hub slidably received in the first aperture (20),
a flexible tube (60) coupled to the proximal end of the hub slidably received in the second aperture (20'),
a cover (62) removably positioned over the needle when in the extended orientation; and
(iv) the guides (G) for guiding the movement of the wings (56), formed in the edges of the sections including a primary recess (66) formed in the upper edge (32) of the lower section between a forward end proximal of the upwardly extending flange projection (38) and a rearward end distal of the male and female projections (42,44),
a forward recess in the lower edge of the upper section proximal of the forward end of the primary recess, and
an abutment surface formed in the lower edge of the upper section distal of the rearward end of the primary recess, wherein
the wings (56) of the needle assembly (48) slidably placed in the housing assembly (14) are adapted to extend laterally from the hub (50) through the primary recess (66) at an extended orientation with the majority of the needle extending distal of the main body portion (16) and with the forward recess (68) assisting in retaining the needle in the extended orientation (EO), and
the wings are adapted to extend laterally from the hub through the primary recess (66) at a retracted orientation (RO) with the entirety of the needle totally within the main body portion (16) and with the abutment surface (70) assisting in retaining the needle in the retracted orientation (RO), the needle assembly (48) including the needle (58) adapted to be moved from the extended orientation (EO) to the retracted orientation (RO) by a user sliding the wings within the primary recess thereby enabling:
when a used winged needle (58) is pulled out from a patient, the flange projection (38) is capable of being pushed forward using the index finger of a hand on the flange projection while both wings (56) are capable of being held or drawn backward using the thumb and middle finger of the same hand.

2. The system as set forth in claim 1, wherein the system has a chamfered constitution in that most edges and corners are chamfered and have round chamfered finishing (r1, r2, r3, r4, r5, r6, r7,...).

## Patentansprüche

1. Nadelstichschutzsystem (10) zum umgehenden Abschirmen einer Nadel einer Flügelnadel, wenn sie unter Verwendung von nur einer Hand von einem Patienten zurückgezogen wird, wobei das Abschirmen auf eine sichere, bequeme und ökonomische Weise erfolgt, wobei das System folgendes aufweist:
(i) eine Gehäuseanordnung (14),
(ii) ein Handhabungselement (H),
(iii) eine Nadelanordnung (48), und
(iv) Führungen (G),
die in Kombination arbeiten,
wobei
(i) die Gehäuseanordnung (14) zum Unterbringen der in ihr verschiebbar platzierten Nadelanordnung (48) folgendes aufweist:
einen Hauptkörperteilbereich (16) mit einem distalen Ende (18), das mit einer ersten Öffnung (20) ausgebildet ist, und einem proximalen Ende (22), das mit einer zweiten Öffnung (20') ausgebildet ist, und mit einem zylindrischen Umfang in einer allgemein rechteckförmigen Außenquerschnittskonfiguration über den Großteil seiner Länge zwischen dem distalen und dem proximalen Ende (18, 22),
wobei der Hauptkörperteilbereich (16) einen halbzylindrischen oberen Abschnitt (I) mit einem unteren Rand (28) und einen halbzylindrischen unteren Abschnitt (II) mit einem oberen Rand (32) hat,
ein Paar von flexiblen Gelenkteilbereichen (34) mit oberen Enden, die mit dem oberen Abschnitt (I) an dem distalen Ende (18) gekoppelt sind, und unteren Enden, die mit dem unteren Abschnitt (II) an dem distalen Ende (18) gekoppelt sind,
wobei die Gelenkteilbereiche (34) dazu geeignet sind, eine Bewegung der Hälften zwischen einer geschlossenen Betriebsorientierung und einer offenen Nichtbetriebsorientierung zuzulassen,
wobei die geschlossene Betriebsorientierung derart ist, dass die oberen und unteren Ränder in einer horizontalen Ebene in Kontakt sind, um den Hauptkörperteilbereich (16) in einen Zylinder mit einem kreisförmigen Querschnitt auszubilden,
wobei die offene Nichtbetriebsorientierung derart ist, dass die oberen und unteren Ränder nicht in Kontakt und voneinander beabstandet sind, wobei die Gelenkteilbereiche (34) auf entgegengesetzten Seiten der ersten Öffnung lokalisiert sind;
(ii) die Handhabungselemente (H) zum Versehen des Systems mit guter Handhabungsfähigkeit eines Zulassens einer einhändigen Nadelzurückziehprozedur mit dem Hauptkörperteilbereich (16) ausgebildet sind,
wobei die Handhabungselemente (H) einen sich nach oben erstreckenden plattenartig geformten Flanschvorsprung (38) enthalten, der an dem halbzylindrischen oberen Abschnitt (26) im Wesentlichen koplanar zu dem distalen Ende (18) des Hauptkörperteilbereichs (16) lokalisiert ist,
wobei der plattenartig geformte Flanschvorsprung (38) zwei Seiten hat, die eine nach vorn orientierte Fläche (38') auf der distalen Seite und eine nach hinten orientierte Fläche (38") auf der proximalen Seite aufweist,
wobei die nach vorn orientierte Fläche des Flanschvorsprungs im Wesentlichen vertikal und koplanar zu dem distalen Ende (18) ist, und
wobei die nach hinten orientierte Fläche unter einem Winkel nach oben geneigt ist, der größer als ein rechter Winkel (90°) und kleiner als 120° ist, oder 90°< θ1 < 120°, wobei θ1 der Winkel ist, der zwischen der nach hinten orientierten Fläche und einer Bodenlinie L gebildet ist,
wobei die Handhabungselemente (H) eine Vielzahl von sich lateral erstreckenden rutschfesten Vertiefungen (40) enthalten, die an dem oberen Abschnitt nahe dem sich nach oben erstreckenden Flanschvorsprung (38) lokalisiert sind,
wobei die Handhabungselemente (H) auch sich nach außen erstreckende Buchsenvorsprünge (42) enthalten, die an dem oberen Abschnitt benachbart zu dem proximalen Ende lokalisiert sind, mit Steckervorsprüngen (44), die sich von dem unteren Abschnitt in kooperierbarer Beziehung zu dem Buchsenvorsprung (42) nach oben erstrecken, zum loslösbaren Sichern der Abschnitte miteinander in der geschlossenen Orientierung (CO);
(iii) die Nadelanordnung (48) folgendes enthält:
eine zylindrische hohle Nabe (50) mit einem distalen Ende (52) und einem proximalen Ende (54), die verschiebbar innerhalb der Gehäuseanordnung (14) positioniert ist,
wobei die Nabe (50) flache, sich lateral erstreckende Flügel (56) hat, und eine Nadel (58), die mit dem distalen Ende (54) der Nabe gekoppelt ist, in der ersten Öffnung (20) verschiebbar aufgenommen,
ein flexibles Rohr (60), das mit dem proximalen Ende der Nabe gekoppelt ist, verschiebbar aufgenommen in der zweiten Öffnung (20'),
eine Abdeckung (62), die über der Nadel entfernbar positioniert ist, wenn sie in der ausgefahrenen Orientierung ist; und
(iv) die Führungen (G) zum Führen der Bewegung der Flügel (56), die in den Rändern der Abschnitte ausgebildet sind, eine primäre Vertiefung (66) enthalten, die in dem oberen Rand (32) des unteren Abschnitts zwischen einem vorwärtigen Ende proximal von dem sich nach oben erstreckenden Flanschvorsprung (38) und einem rückwärtigen Ende distal von den Stecker- und Buchsenvorsprüngen (42, 44) ausgebildet ist,
eine vorwärtige Vertiefung in dem unteren Rand des oberen Abschnitts proximal zu dem vorwärtigen Ende der primären Vertiefung, und
eine Widerlageroberfläche, die in dem unteren Rand des oberen Abschnitts distal von dem rückwärtigen Ende der primären Vertiefung ausgebildet ist, wobei
die Flügel (56) der Nadelanordnung (48), die in der Gehäuseanordnung (14) verschiebbar platziert sind, dazu geeignet sind, sich von der Nabe (50) durch die primäre Vertiefung (66) bei einer ausgefahrenen bzw. ausgestreckten Orientierung lateral zu erstrecken, wobei sich der Großteil der Nadel distal von dem Hauptkörperteilbereich (16) erstreckt und wobei die vorwärtige Vertiefung (68) beim Beibehalten der Nadel in der ausgefahrenen Orientierung (EO) hilft, und
die Flügel dazu geeignet sind, sich bei einer zurückgezogenen Orientierung (RO) von der Nabe durch die primäre Vertiefung (66) lateral zu erstrecken, wobei die Gesamtheit der Nadel insgesamt innerhalb des Hauptkörperteilbereichs (16) ist und wobei die Widerlageroberfläche (70) beim Beibehalten der Nadel in der zurückgezogenen Orientierung (RO) hilft, wobei die Nadelanordnung (48) die Nadel (58) enthält, die dazu geeignet ist, durch einen Anwender, der die Flügel innerhalb der primären Vertiefung verschiebt, von der ausgefahrenen Orientierung (EO) zu der zurückgezogenen Orientierung (RO) bewegt zu werden, um dadurch folgendes zu ermöglichen:
wenn eine benutzte Flügelnadel (58) aus einem Patienten herausgezogen wird, kann der Flanschvorsprung (38) unter Verwendung des Zeigefingers einer Hand an dem Flanschvorsprung nach vorn geschoben werden, während beide Flügel (56) unter Verwendung des Daumens und des Mittelfingers derselben Hand gehalten oder zurückgezogen werden können.

2. System nach Anspruch 1, wobei das System eine abgeschrägte Konfiguration diesbezüglich hat, dass die meisten Ränder und Ecken abgeschrägt sind und eine gerunde abgeschrägte Endbearbeitung (r1, r2, r3, r4, r5, r6, r7,...) haben.

## Revendications

1. Système anti-piqûre accidentelle d'aiguille (10) utilisé pour protéger l'aiguille d'une aiguille papillon immédiatement après son retrait du patient, à l'aide d'une seule main, le protecteur étant sûr, pratique et économique, ledit système comprenant,
(i) un ensemble logement (14),
(ii) un ensemble de manipulation (H),
(iii) un ensemble aiguille (48), et
(iv) des guides (G)
utilisés en combinaison
dans lequel
(i) l'ensemble logement (14) dans lequel vient se glisser l'ensemble aiguille (48), comportant,
une partie de corps principal (16) dont l'extrémité distale (18) comprend une première ouverture (20) et dont l'extrémité proximale (22) comprend une seconde ouverture (20') et une extension cylindrique dont la configuration de coupe transversale extérieure est globalement rectangulaire sur la plus grande partie de sa longueur, entre l'extrémité distale et l'extrémité proximale (18, 22),
la partie de corps principal (16) ayant une section supérieure semi-cylindrique (I) avec un bord inférieur (28) et une section inférieure semi-cylindrique (II) avec un bord supérieur (32),
deux parties articulées flexibles (34) dont les extrémités supérieures sont couplées à la section supérieure (I) au niveau de l'extrémité distale (18) et dont les extrémités inférieures sont couplées à la section inférieure (II) au niveau de l'extrémité distale (18),
les parties articulées (34) étant adaptées pour permettre le mouvement des moitiés entre un sens opérant fermé et un sens inopérant ouvert,
le sens opérant fermé étant avec les bords, supérieur et inférieur, en contact sur le plan horizontal, afin de former la partie du corps principal (16) ayant la forme d'un cylindre présentant une coupe transversale circulaire,
le sens inopérant ouvert étant, avec les bords, supérieur et inférieur, sans contact et espacés l'un de l'autre, les parties articulées (34) étant situées sur les côtés opposés de la première ouverture ;
(ii) les éléments de manutention (H) permettant la bonne manipulation du système et la capacité de procéder au retrait de l'aiguille d'une seule main, formés sur la partie du corps principal (16)
les éléments de manutention (H) incluant une collerette en forme de plaque se prolongeant vers le haut (38) située sur la section semi-cylindrique supérieure (26) essentiellement coplanaire avec l'extrémité distale (18) de la partie du corps principal (16),
la collerette en forme de plaque (38) comportant deux côtés comprenant une face dirigée vers l'avant (38') du côté distal et une face dirigée vers l'arrière (38") du côté proximal,
la face dirigée vers l'avant de la collerette est essentiellement verticale et coplanaire par rapport à l'extrémité distale (18) et
la face dirigée vers l'arrière est inclinée vers le haut, selon un angle supérieur à un angle droit (90°) et inférieur à 120° ou 90° <θ1<120°, soit 61 est l'angle formé entre la face dirigée vers l'arrière et la ligne de plancher L,
les éléments de manutention (H) incluant une pluralité de creux antidérapants se prolongeant latéralement (40), situés sur l'extrémité proximale de la section supérieure de la collerette se prolongeant vers le haut (38),
les éléments de manutention (H) incluant également des saillies femelles se prolongeant vers l'extérieur (42), situées sur la section supérieure adjacente à l'extrémité proximale comportant des saillies mâles (44) se prolongeant vers le haut depuis la section inférieure et pouvant coopérer avec la saillie femelle (42) afin de fixer de façon séparable les sections ensemble dans le sens fermé (CO) ;
(iii) l'ensemble aiguille (48) comprenant,
une embase creuse cylindrique (50) avec une extrémité distale (52) et une extrémité proximale (54) positionnées de façon à pouvoir coulisser dans l'ensemble logement (14),
l'embase (50) comportant des ailettes plates se prolongeant latéralement (56), une aiguille (58) couplée à l'extrémité distale (54) de l'embase reçue de façon à pouvoir coulisser dans la première ouverture (20),
un tube flexible (60) couplé à l'extrémité proximale de l'embase réceptionnée de façon à pouvoir coulisser dans la deuxième ouverture (20'),
un capuchon amovible (62) positionné sur l'aiguille dans le sens déployé, et
(iv) les guides (G) pour guider le mouvement des ailettes (56) formées sur les bords des sections comprenant un premier creux (66) formé sur le bord supérieur (32) de la section inférieure entre une extrémité avant proximale de la collerette se prolongeant vers le haut (38) et une extrémité arrière distale des saillies mâles et femelles (42, 44),
un creux avant formé sur le bord inférieur de la section supérieure proximale de l'extrémité avant du premier creux, et
une surface de butée formée sur le bord inférieur de la section supérieure distale de l'extrémité dirigée vers l'arrière du premier creux, dans laquelle,
les ailettes (56) de l'ensemble aiguille (48) positionnée de façon à pouvoir coulisser dans l'ensemble logement (14) sont adaptées pour se prolonger latéralement à partir de l'embase (50) à travers le premier creux (66) dans le sens du déploiement, la plus grande partie de l'aiguille se prolongeant distalement par rapport à la partie du corps principal (16) et le creux avant (68) participant à la rétention de l'aiguille dans le sens du déploiement (EO), et
les ailettes sont adaptées pour se déployer latéralement depuis l'embase, à travers le premier creux (66) dans le sens de rétraction (RO) l'intégralité de l'aiguille se trouvant complètement à l'intérieur de la partie du corps principal (16) et la surface de butée (70) participant à la rétention de l'aiguille dans le sens rétracté (RO), l'ensemble aiguille (48) incluant l'aiguille (58) adaptée pour pouvoir passer du sens déployé (EO) au sens rétracté (RO) par un utilisateur faisant coulisser les ailettes à l'intérieur du premier creux ce qui permet ainsi :
lorsqu'une aiguille papillon (58) usagée est retirée d'un patient, la collerette (38) peut être poussée vers l'avant avec l'index d'une seule main placé sur la collerette, tandis que les deux ailettes (56) peuvent être maintenues ou tirées vers l'arrière en utilisant le pouce et le majeur de la même main.

2. Système tel que décrit dans la revendication 1, dans lequel le système a une forme chanfreinée dans la mesure où la majorité des bords et des coins est chanfreinée et comporte une finition chanfreinée arrondie (r1, r2, r3, r4, r5, r6, r7,...).
